# EUROPEAN PATENT SPECIFICATION

(11) **EP 3 383 483 B1**
(45) Date of publication and mention of the grant of the patent: **21.04.2021**
(21) Application number: 16869351.3
(22) Date of filing: 28.11.2016
(51) Int. Cl.: A61N 1/36, A61N 1/378, A61N 1/372, H02J 50/10, A61N 1/08

(54) **DISPOSABLE GASTROINTESTINAL IMPLANTABLE STIMULATOR**
GASTROINTESTINALER IMPLANTIERBARER EINWEGSTIMULATOR
STIMULATEUR IMPLANTABLE GASTRO-INTESTINAL JETABLE

(30) Priority: 29.11.2015 US 201562260624 P
(43) Date of publication of application: 10.10.2018
(73) Proprietor: The Regents of the University of California, Oakland, CA 94607 (US)
(72) Inventor: LO, Yi-Kai, Los Angeles, California 90095-1600 (US); LIU, Wentai, Los Angeles, California 90095-1600 (US)
(74) Representative: Richards, John
(86) International application number: PCT/US2016/063886
(87) International publication number: WO 2017/091828

(56) References cited:
- WO-A1-2010/005482
- CN-B- 102 500 057
- US-A1- 2005 137 644
- US-A1- 2005 209 653
- US-A1- 2008 300 654
- US-A1- 2008 300 654
- US-A1- 2008 306 359
- US-A1- 2008 306 359
- US-A1- 2009 105 784
- US-A1- 2015 217 120
- US-B1- 6 631 296
- US-B2- 8 944 993

## Description

### BACKGROUND

### 1. Technical Field

This description pertains generally to medical implants, and more particularly to medical implants for treating post operative ileus.

### 2. Background Discussion

Post-operative ileus (POI) leads to the inflammation of the bowel wall that occurs following abdominal surgery and its economic impact is estimated to be between $3/4 billion and $1 billion per year in the United States. Patients with POI manifest abdominal pain, nausea, vomiting, as well as the inability of coordinated propulsive mobility while the current treatment is restricted to the spontaneous recovery of the patient.

POI is not only limited to patients receiving abdominal surgery. There are patients receiving open-heart surgery also reporting symptoms similar to POI, possibly because the sympathetic and parasympathetic nerves governing the GI tack are affected by the surgery.

US 6,631,296 discloses a voltage converter for use within small implantable electrical devices, such as a microstimulator, which uses a coil, instead of capacitors, to provide a voltage step up and step down function. The output voltage is controlled, or adjusted, through duty-cycle modulation. Good efficiencies are achieved for virtually any voltage within the compliance range of the converter. In accordance with one aspect applicable to implantable devices having an existing RF coil through which primary or charging power is provided, the existing RF coil is used in a time-multiplexing scheme to provide both the receipt of the RF signal and the voltage conversion function. This minimizes the number of components needed within the device, and thus allows the device to be packaged in a smaller housing, or frees up additional space within an existing housing for other circuit components. In accordance with another aspect, the voltage up/down converter circuit is controlled by a pulse width modulation (PWM) low power control circuit. Such operation allows high efficiencies over a wide range of output voltages and current loads. There is thus provided a voltage converter circuit for use within an implantable device that is compact, efficient, and provides a wide range of output voltages and currents.

### BRIEF SUMMARY

The invention is defined by independent claim 1. A primary premise of the system and methods disclosed herein is that electrical stimulation in the vagus nerve (VN) reduces the level of tumor necrosis factor (TNF), indicating the decrease of inflammation. Thus, embodiments of the present technology are configured to treat POI through electrophysiological intervention by stimulating the bowel wall where the nerve ending of VN is located. For the therapeutic treatment of POI, the device performing stimulation is small and easily/conveniently removable after a course of POI treatment.

Accordingly, one aspect of the present technology is an implant that may be positioned inside the gastrointestinal (GI) tract through laparotomy or laparoscopic surgery. The implant may be secured in place using a biodegradable glue or biodegradable suture (e.g. catgut that dissolves in a few days), and is naturally expelled from the body with bowel movement after a certain period of time. By way of example, and not of limitation, the GI implant comprises a coil that receives power from, and sends the recorded physiological information to, an external device through wireless inductive coupling.

Further aspects of the technology will be brought out in the following portions of the specification, wherein the detailed description is for the purpose of fully disclosing preferred embodiments of the technology without placing limitations thereon.

### BRIEF DESCRIPTION OF THE SEVERAL VIEWS

### OF THE DRAWING(S)

The technology described herein will be more fully understood by reference to the following drawings which are for illustrative purposes only:
FIG. 1 shows a schematic diagram of the gastrointestinal (GI) stimulation system of the present description, with disposable GI implant installed inside the GI tract and external stimulator disposed adjacent a patient's abdominal wall.
FIG. 2 shows a schematic illustration of possible placement locations for the disposable GI implant of FIG. 1 within the GI tract.
FIG. 3 shows a detailed schematic diagram of the disposable GI implant of FIG. 1.
FIG. 4 is a schematic illustration of exemplary stimulation parameters control and chip powering in accordance with a method of the present technology.
FIG. 5 is a schematic illustration of an exemplary modulation scheme for reverse data link in accordance with a method of the present technology.
FIG. 6 shows a schematic diagram of the external control device for use with the gastrointestinal (GI) stimulation system of the present description.
FIG. 7 is a schematic diagram of a processing module for implementation of any of the systems or methods of FIG. 1 through FIG. 6.

### DETAILED DESCRIPTION

The technology that is described herein is based on systems and methods for implementation of a disposable miniaturized implant for treatment of Post-Operative Ileus (POI). The primary function of the implant is to provide electrical stimulation to the part of bowel going through surgery to expedite the healing process while recording the smooth muscle activities simultaneously. Disposability of the implant is a key feature, as patients with POI would be less willing to undergo another surgery to remove the device.

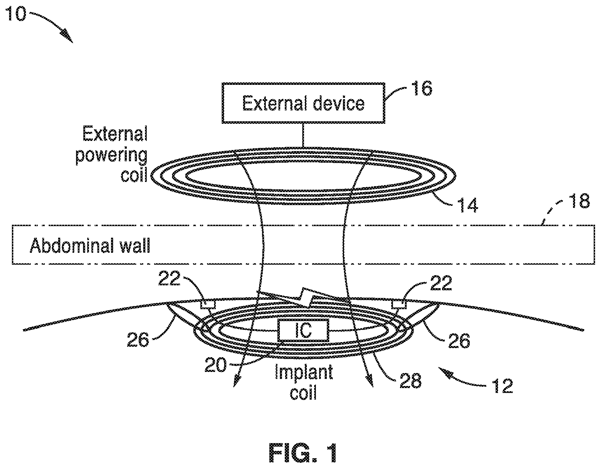

FIG. 1 shows a schematic diagram of the gastrointestinal (GI) stimulation system 10 of the present description, with a disposable GI implant 12 installed inside the GI tract and an external stimulator 16 disposed adjacent a patient's abdominal wall 18.

In a preferred embodiment, the GI implant 12 receives power from, and sends the recorded physiological information to the external device 16, through wireless inductive coupling generated via an implant coil 28 coupled to an IC 20 of implant 12 and external powering coil 14 coupled to the external device 16.

In one embodiment, the implant 12 and the stimulation/recording electrodes 22/24 are adhered on the inner wall of the GI tract by suturing or gluing 26. The suture or glue 26 is configured to be gradually degraded inside the GI tract such that the implant 12 is propelled out of the body with the stool in days or weeks.

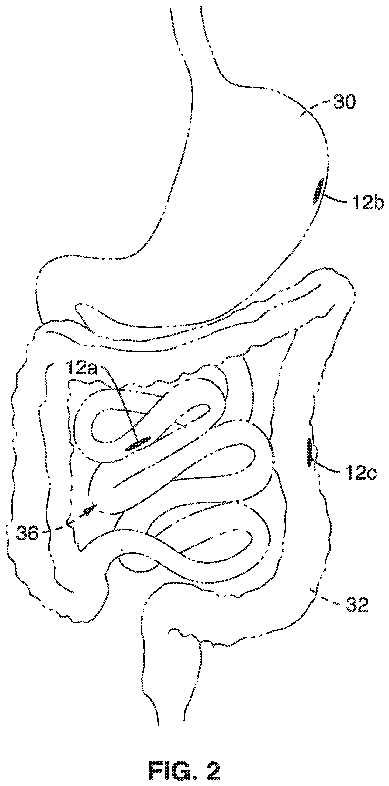

FIG. 2 shows a schematic illustration of possible placement locations for the disposable GI implant 12 within the GI tract. For example, implant locations may comprise one or more of a small intestinal implant 12a with a location of the small intestines 36, stomach implant 12b in the stomach 30, and large intestine implant 12c at a location of the large intestines 32.

The implant 12 can be placed through laparotomy or laparoscopic surgery. The implant 12 is preferably secured in place using a biodegradable glue or biodegradable suture 26 (e.g. catgut that dissolves in a few days). The implant 12 would then be naturally expelled from the body with bowel movement after a certain period of time.

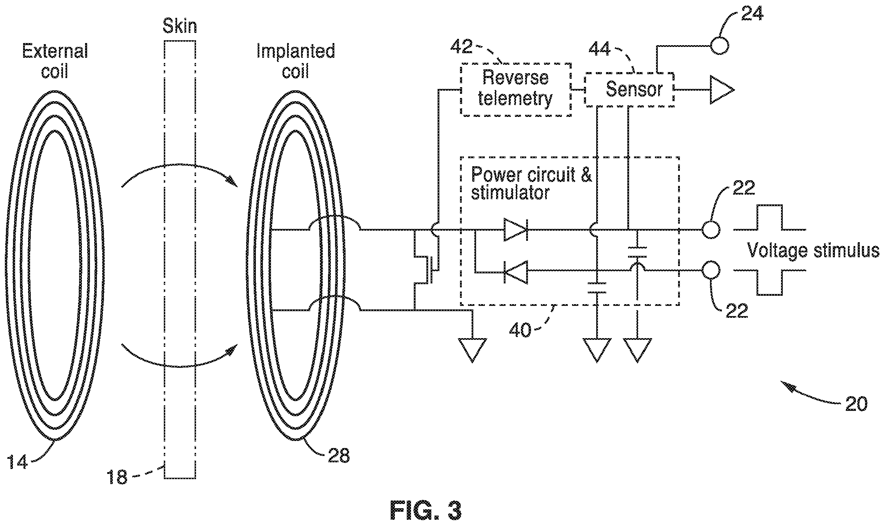

FIG. 3 shows a detailed schematic diagram of the disposable GI implant 12. In the embodiment shown, the implant comprises five primary components as follows: a power circuit and stimulator module 40, a reverse telemetry module 42, and sensor 44, one or more of which may be disposed on integrated circuit (IC) 20, implantable coil 28, and stimulation/recording electrodes 22/24. As shown in the schematic diagram of FIG. 3, a half-wave rectifier is used to retrieve the induced AC voltage from the implant coil 28 and convert it to DC voltage. The regulated DC voltages are used to perform bipolar electrical stimulation via the module 40 and electrodes 22.

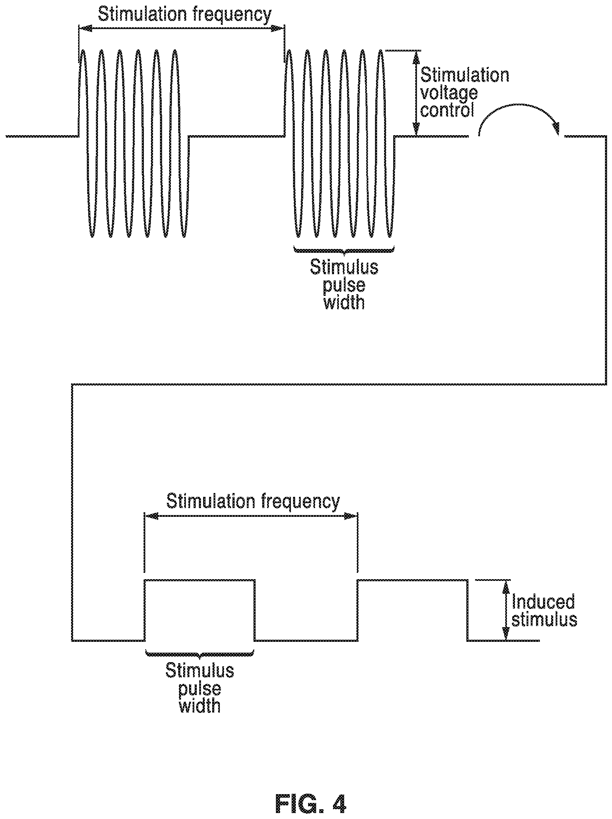

FIG. 4 is a schematic illustration of exemplary stimulation parameters for control and chip powering in accordance with a method of the present technology. Instead of transmitting a continuous power signal to the implant 12, the power signal at the external coil is modulated. Thus, in the implant 12, the power circuit and stimulator module 40 produces constant voltage stimulus, whose frequency/pulse width/intensity is determined by its received power signal. The sensor 44 then measures the stimulation voltage and the physiological signal of the smooth muscle via one or more electrodes 24. The reverse telemetry circuits 42 subsequently sends the signal through the same coil 28 using load-shift keying that shorts the coil when bit 1 is transmitted.

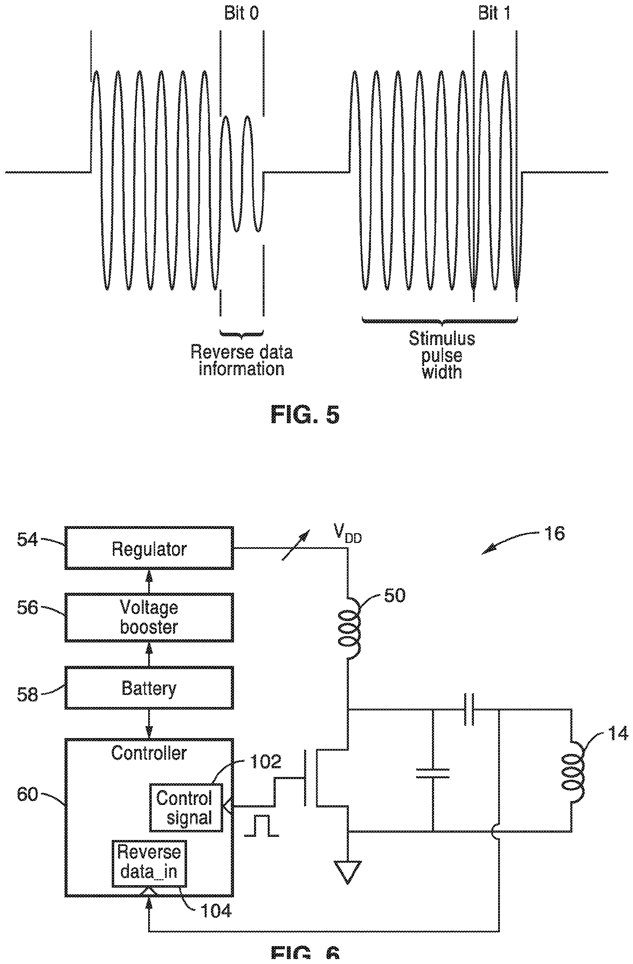

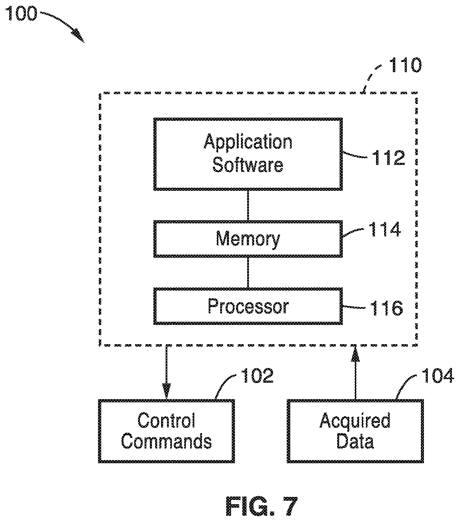

FIG. 5 is a schematic illustration of an exemplary modulation scheme for a reverse data link in accordance with a method of the present technology. The stimulation intensity is mainly determined by the induced voltage at the implanted coil 28 (i.e. amplitude at the primary coil). When transmitting the sensed stimulus intensity and the physiological signal (e.g. acquired data signal 104, FIG. 7) through the power coil 28, the induced voltage is attenuated as its loading condition changes. In one embodiment, the transmitted data 104 is inserted at the very end of each power signal, such that the power signal generated by the primary coil 28 is less influenced. Through the reverse link, a bit 0 can be recognized when there is drop in the amplitude of the induced voltage and vice versa. The length of either bit 1 or bit 0 is varied.

FIG. 6 shows a schematic diagram of the external control device 16 for use with the gastrointestinal (GI) stimulation system 10 of the present description. In the embodiment shown, the external device 16 comprises a power transmitter 50/14 that sends the power signal to the implant 12; a controller 60 that controls the stimulation parameters (i.e. pulse/frequency and intensity); a battery 58; a voltage booster 56 to increase the battery voltage, and a regulator 54 that powers the power transmitter. In the embodiment shown in FIG. 6, a class-E power amplifier is used as an example, but different topology can be employed. The controller 60 senses the information and tunes the stimulation parameters. Note that stimulation intensity is adjusted by varying V_{DD} through the regulator 54.

FIG. 7 is a schematic diagram of a processing module 100 for implementation of any of the systems or methods of FIG. 1 through FIG. 6. Processing module 100 may be implemented for operation of the external device 16, controller 60 of the implant 12, or both. Processing module 100 comprises application programming 112 that may be stored in memory 114 and executable on processor 116 for acquiring sensor data 104 and generating control signals 102. Processing module 100 may comprise a computer 110 or other form of hardware.

Embodiments of the present technology may be described with reference to flowchart illustrations of methods and systems according to embodiments of the technology, and/or algorithms, formulae, or other computational depictions, which may also be implemented as computer program products. In this regard, each block or step of a flowchart, and combinations of blocks (and/or steps) in a flowchart, algorithm, formula, or computational depiction can be implemented by various means, such as hardware, firmware, and/or software including one or more computer program instructions embodied in computer-readable program code logic. As will be appreciated, any such computer program instructions may be loaded onto a computer, including without limitation a general purpose computer or special purpose computer, or other programmable processing apparatus to produce a machine, such that the computer program instructions which execute on the computer or other programmable processing apparatus create means for implementing the functions specified in the block(s) of the flowchart(s).

Accordingly, blocks of the flowcharts, algorithms, formulae, or computational depictions support combinations of means for performing the specified functions, combinations of steps for performing the specified functions, and computer program instructions, such as embodied in computer-readable program code logic means, for performing the specified functions. It will also be understood that each block of the flowchart illustrations, algorithms, formulae, or computational depictions and combinations thereof described herein, can be implemented by special purpose hardware-based computer systems which perform the specified functions or steps, or combinations of special purpose hardware and computer-readable program code logic means.

Furthermore, these computer program instructions, such as embodied in computer-readable program code logic, may also be stored in a computer-readable memory that can direct a computer or other programmable processing apparatus to function in a particular manner, such that the instructions stored in the computer-readable memory produce an article of manufacture including instruction means which implement the function specified in the block(s) of the flowchart(s). The computer program instructions may also be loaded onto a computer or other programmable processing apparatus to cause a series of operational steps to be performed on the computer or other programmable processing apparatus to produce a computer-implemented process such that the instructions which execute on the computer or other programmable processing apparatus provide steps for implementing the functions specified in the block(s) of the flowchart(s), algorithm(s), formula(e), or computational depiction(s).

It will further be appreciated that the terms "programming" or "program executable" as used herein refer to one or more instructions that can be executed by a processor to perform a function as described herein. The instructions can be embodied in software, in firmware, or in a combination of software and firmware. The instructions can be stored local to the device in non-transitory media, or can be stored remotely such as on a server, or all or a portion of the instructions can be stored locally and remotely. Instructions stored remotely can be downloaded (pushed) to the device by user initiation, or automatically based on one or more factors. It will further be appreciated that as used herein, that the terms processor, computer processor, central processing unit (CPU), and computer are used synonymously to denote a device capable of executing the instructions and communicating with input/output interfaces and/or peripheral devices.

## Claims

1. An implantable apparatus for stimulating tissue, comprising:
an implantable coil (28);
a power circuit and stimulator module (40) comprising a half-wave rectifier configured to convert an induced AC voltage on the implantable coil into a DC voltage, wherein the power circuit and stimulator module (40) is configured to produce a constant voltage stimulus whose one or more stimulation parameters being frequency, pulse width or intensity are determined by a power signal received at said implantable coil (28) from an external device (16);
stimulus electrodes (22) connected to the power circuit and stimulator module (40), wherein said stimulus electrodes are configured to perform bipolar electrical stimulation using the voltage stimulus produced by the power circuit and stimulator module;
a reverse telemetry module (42) connected to the implantable coil;
a sensor (44) connected to the reverse telemetry module; and
at least one recording electrode (24) connected to the sensor;
wherein the implantable coil (28) is configured to couple to the external device through a wireless inductive coupling allowing the external device to control the one or more stimulation parameters when applying the voltage stimulus by the implantable apparatus at a treatment location in a body tissue through the stimulus electrodes (22); and
wherein the sensor (44) is configured to measure one or more of the intensity of the applied voltage stimulus and a physiological signal received from the body tissue for transmission as measurements by the reverse telemetry module (42) to the external device.

2. The implantable apparatus of claim 1, wherein the reverse telemetry module (42) is configured to transmit the measurements to the external device through the wireless inductive coupling.

3. The implantable apparatus of claim 2, wherein the reverse telemetry module (42) is configured to transmit the measurements by load shift keying that shorts the implantable coil when a bit 1 is to be transmitted and drops the amplitude of a voltage induced on the implantable coil when a bit 0 is to be transmitted.

4. The implantable apparatus of claim 3, wherein the reverse telemetry module is configured to insert the bit to be transmitted at the end of the power signal.

5. The implantable apparatus of claim 1, wherein the physiological signal is a physiological signal of a smooth muscle.

6. The implantable apparatus of claim 1, wherein said implantable apparatus is configured for treatment of Post-Operative Ileus (POI) to provide electrical stimulation to a part of a bowel going through surgery to expedite a healing process.

7. The implantable apparatus of claim 1, wherein the implantable apparatus is configured to be anchored to an internal wall of the body using biodegradable sutures (26) or biodegradable glue (26).

8. The implantable apparatus of claim 7,
wherein the implantable apparatus is configured to be installed at a gastrointestinal treatment location; and
wherein the implantable apparatus is configured to be naturally expelled through a bowel after degradation of the biodegradable sutures (26) or biodegradable glue (26).

9. The implantable apparatus of claim 1, wherein said implantable apparatus is configured for treating Post-operative ileus (POI), which can lead to inflammation of a bowel wall, said implantable apparatus being configured for electrophysiological intervention by stimulating the bowel wall at a location of vagus nerve (VN) endings.

10. The implantable apparatus of claim 1, wherein said implantable apparatus is configured as a implant selected from a group of gastrointestinal implants consisting of a small intestine implant (12a), a stomach implant (12b), and a large intestine implant (12c).

11. The implantable apparatus of claim 1, wherein said implantable apparatus is a disposable miniaturized implant which is sufficiently small allowing implantation through laparotomy or laparoscopic surgery, and which is configured to be expelled naturally after a certain period of time.

12. The implantable apparatus of claim 1, wherein one or more of the power circuit and stimulator module, the reverse telemetry module, and the sensor are disposed on an integrated circuit (IC) within the implantable apparatus.

## Patentansprüche

1. Implantierbare Vorrichtung zur Stimulation von Gewebe, wobei die Vorrichtung folgendes umfasst:
eine implantierbare Spule (28);
ein Hauptstromkreis- und Stimulatormodul (40), das einen Halbwellengleichrichter umfasst, der so gestaltet ist, dass er eine induzierte Wechselstromspannung an der implantierbaren Spule in eine Gleichstromspannung umwandelt, wobei das Hauptstromkreis- und Stimulatormodul (40), so gestaltet ist, dass es einen konstanten Spannungsstimulus erzeugt, dessen ein oder mehreren Stimulationsparameter, Frequenz, Impulsbreite oder Intensität, durch ein Leistungssignal bestimmt werden, das von einer externen Vorrichtung (16) an der implantierbaren Spule (28) empfangen wird;
Stimulationselektroden (22), die mit dem Hauptstromkreis- und Stimulatormodul (40) verbunden sind,
wobei die Stimulationselektroden so gestaltet sind, dass sie eine bipolare elektrische Stimulation unter Verwendung des durch das Hauptstromkreis- und Stimulatormodul bereitgestellten Spannungsstimulus ausführen;
ein umgekehrtes Telemetriemodul (42), das mit der implantierbaren Spule verbunden ist;
einen Sensor (44), der mit dem umgekehrten Telemetriemodul verbunden ist; und
mindestens eine Aufzeichnungselektrode (24), die mit dem Sensor verbunden ist;
wobei die implantierbare Spule (28) so gestaltet ist, dass sie eine Kopplung mit der externen Vorrichtung über eine drahtlose induktive Kopplung vorsieht, so dass die externe Vorrichtung den einen oder die mehreren Stimulationsparameter steuern kann, wenn der Spannungsstimulus durch die implantierbare Vorrichtung an einer Behandlungsstelle in einem Körpergewebe durch die Stimulationselektroden (22) zugeführt wird; und
wobei der Sensor (44) so gestaltet ist, dass eines oder mehrere der folgenden misst: die Intensität des zugeführten Spannungsstimulus und/oder ein physiologisches Signal, das von dem Körpergewebe empfangen wird, zur Übermittlung als Messungen durch das umgekehrte Telemetriemodul (42) an die externe Vorrichtung.

2. Implantierbare Vorrichtung nach Anspruch 1, wobei das umgekehrte Telemetriemodul (42) so gestaltet ist, dass es die Messungen über die drahtlose induktive Kopplung an die externe Vorrichtung übermittelt.

3. Implantierbare Vorrichtung nach Anspruch 2, wobei das umgekehrte Telemetriemodul (42) so gestaltet ist, dass es die Messungen durch Load-Shift-Keying übermittelt, wobei die implantierbare Spule kurzgeschlossen wird, wenn ein Bit 1 übermittelt werden soll, und wobei die Amplitude einer an der implantierbaren Spule induzierten Spannung gesenkt wird, wenn ein Bit 0 übermittelt werden soll.

4. Implantierbare Vorrichtung nach Anspruch 3, wobei das umgekehrte Telemetriemodul so gestaltet ist, dass es das zu übermittelnde Bit am Ende des Leistungssignals einfügt.

5. Implantierbare Vorrichtung nach Anspruch 1, wobei das physiologische Signal ein physiologisches Signal eines glatten Muskels ist.

6. Implantierbare Vorrichtung nach Anspruch 1, wobei die implantierbare Vorrichtung gestaltet ist für die Behandlung von postoperativem Ileus (POI), um einen Teil eines Darms elektrisch zu stimulieren, der einem chirurgischen Eingriff unterzogen wird, um einen Heilprozess zu beschleunigen.

7. Implantierbare Vorrichtung nach Anspruch 1, wobei die implantierbare Vorrichtung gestaltet ist zur Verankerung an einer Innenwand des Körpers unter Verwendung biologisch abbaubarer Nähte (26) oder von biologisch abbaubarem Klebstoff (26).

8. Implantierbare Vorrichtung nach Anspruch 7,
wobei die implantierbare Vorrichtung gestaltet ist zur Installation an einem gastrointestinalen Behandlungsort; und
wobei die implantierbare Vorrichtung so gestaltet ist, dass sie auf natürliche Weise aus einem Darm ausgestoßen wird nach dem Abbau der biologisch abbaubaren Nähte (26) oder des biologisch abbaubaren Klebstoffs (26).

9. Implantierbare Vorrichtung nach Anspruch 1, wobei die implantierbare Vorrichtung gestaltet ist für die Behandlung von postoperativem Ileus (POI), der zu einer Entzündung einer Darmwand führen kann, wobei die implantierbare Vorrichtung für eine elektrophysiologische Intervention durch Stimulation der Darmwand an einer Stelle von Enden des Nervus Vagus (NV) gestaltet ist.

10. Implantierbare Vorrichtung nach Anspruch 1, wobei die implantierbare Vorrichtung als ein Implantat gestaltet ist, das aus einer Gruppe von gastrointestinalen Implantaten ausgewählt ist, die ein Dünndarmimplantat (12a), ein Magenimplantat (12b) und ein Dickdarmimplantat (12c) umfasst.

11. Implantierbare Vorrichtung nach Anspruch 1, wobei die implantierbare Vorrichtung ein Miniatur-Einwegimplantat ist, das klein genug ist für eine Implantation durch Laparotomie oder laparoskopische Chirurgie, und das so gestaltet ist, dass es nach einem bestimmten Zeitraum auf natürliche Weise ausgestoßen wird.

12. Implantierbare Vorrichtung nach Anspruch 1, wobei sich eines oder mehrere des Hauptstromkreis- und Stimulatormoduls, des umgekehrten Telemetriemoduls und des Sensors auf einer integrierten Schaltung (IC) in der implantierbaren Vorrichtung befindet bzw. befinden.

## Revendications

1. Appareil implantable pour la stimulation des tissus, comprenant :
une bobine implantable (28) ;
un module stimulateur et circuit d'alimentation (40) comprenant un redresseur à une alternance conçu pour convertir une tension CA induite sur la bobine implantable en une tension CC,
le module stimulateur et circuit d'alimentation (40) étant conçu pour produire un stimulus de tension constante dont au moins un paramètre de stimulation, à savoir la fréquence, la largeur d'impulsion ou l'intensité, est déterminé par un signal d'alimentation reçu au niveau de ladite bobine implantable (28) à partir d'un dispositif externe (16) ;
des électrodes de stimulus (22) connectées au module stimulateur et circuit d'alimentation (40),
lesdites électrodes de stimulus étant conçues pour effectuer une stimulation électrique bipolaire en utilisant le stimulus de tension produit par le module stimulateur et circuit d'alimentation ;
un module de télémétrie inverse (42) connecté à la bobine implantable ;
un capteur (44) connecté au module de télémétrie inverse ; et
au moins une électrode d'enregistrement (24) connectée au capteur ;
la bobine implantable (28) étant conçue pour se coupler au dispositif externe par un couplage inductif sans fil permettant au dispositif externe de commander l'au moins un paramètre de stimulation lors de l'application du stimulus de tension par l'appareil implantable au niveau d'un emplacement de traitement dans un tissu corporel par l'intermédiaire des électrodes de stimulus (22) ; et
le capteur (44) étant conçu pour mesurer au moins une intensité du stimulus de tension appliqué et un signal physiologique reçu du tissu corporel pour transmission en tant que mesures par le module de télémétrie inverse (42) au dispositif externe.

2. Appareil implantable selon la revendication 1, le module de télémétrie inverse (42) étant conçu pour transmettre les mesures au dispositif externe par le biais du couplage inductif sans fil.

3. Appareil implantable selon la revendication 2, le module de télémétrie inverse (42) étant conçu pour transmettre les mesures par modulation par déplacement de charge qui court-circuite la bobine implantable lorsqu'un bit 1 doit être transmis et fait chuter l'amplitude d'une tension induite sur la bobine implantable lorsqu'un bit 0 doit être transmis.

4. Appareil implantable selon la revendication 3, le module de télémétrie inverse étant conçu pour insérer le bit à transmettre à la fin du signal d'alimentation.

5. Appareil implantable selon la revendication 1, le signal physiologique étant un signal physiologique d'un muscle lisse.

6. Appareil implantable selon la revendication 1, ledit appareil implantable étant conçu pour le traitement de l'iléus post-opératoire (POI) afin de fournir une stimulation électrique à une partie de l'intestin qui subit une opération chirurgicale pour accélérer un processus de guérison.

7. Appareil implantable selon la revendication 1, l'appareil implantable étant conçu pour être ancré à une paroi interne du corps à l'aide de sutures biodégradables (26) ou de colle biodégradable (26).

8. Appareil implantable selon la revendication 7,
l'appareil implantable étant conçu pour être installé au niveau d'un emplacement de traitement gastro-intestinal ; et
l'appareil implantable étant conçu pour être expulsé naturellement par l'intestin après dégradation des sutures biodégradables (26) ou de la colle biodégradable (26).

9. Appareil implantable selon la revendication 1, ledit appareil implantable étant conçu pour traiter l'iléus postopératoire (POI), qui peut conduire à l'inflammation d'une paroi intestinale, ledit appareil implantable étant conçu pour une intervention électrophysiologique en stimulant la paroi intestinale au niveau d'un emplacement des terminaisons du nerf vague (VN).

10. Appareil implantable selon la revendication 1, ledit appareil implantable étant conçu comme un implant choisi parmi un groupe d'implants gastro-intestinaux consistant en un implant de l'intestin grêle (12a), un implant de l'estomac (12b) et un implant du gros intestin (12c).

11. Appareil implantable selon la revendication 1, ledit appareil implantable étant un implant miniaturisé jetable qui est suffisamment petit pour permettre une implantation par laparotomie ou chirurgie laparoscopique, et qui est conçu pour être expulsé naturellement après une certaine période de temps.

12. Appareil implantable selon la revendication 1, le module stimulateur et circuit d'alimentation, le module de télémétrie inverse et/ou le capteur étant disposés sur un circuit intégré (IC) à l'intérieur de l'appareil implantable.
